# EUROPÄISCHE PATENTSCHRIFT

(11) **EP 0 659 207 B1**
(45) Veröffentlichungstag und Bekanntmachung des Hinweises auf die Patenterteilung: **24.03.1999**
(21) Anmeldenummer: 93919251.4
(22) Anmeldetag: 03.09.1993
(51) Int. Cl.: C11D 1/835, A61K 7/50, E21B 43/22

(54) **VERWENDUNG VON DETERGENSGEMISCHEN**
USE OF DETERGENT MIXTURES
UTILISATION DE MELANGES DE DETERGENTS

(30) Priorität: 11.09.1992 US 943957
(43) Veröffentlichungstag der Anmeldung: 28.06.1995
(60) Teilanmeldung: 98100877.4
(73) Patentinhaber: Henkel Kommanditgesellschaft auf Aktien, 40191 Düsseldorf (DE)
(72) Erfinder: URFER, Allen, D., Lansdale, PA 19446 (US); PONSATI OBIOLS, Oriol, E-08025 Barcelona (ES); BONASTRE, Nuria, E-08210 Barbera del Vallès (ES)
(86) Internationale Anmeldenummer: EP9302376
(87) Internationale Veröffentlichungsnummer: WO9406899

(56) Entgegenhaltungen:
- EP-A- 0 080 855
- EP-A- 0 385 562
- EP-A- 0 387 064
- EP-A- 0 547 722
- WO-A-86/05509
- WO-A-87/02050
- WO-A-92/19693
- WO-A-92/22622
- WO-A-93/03130
- WO-A-93/10748

## Beschreibung

### Gebiet der Erfindung

Die Erfindung betrifft die Verwendung von Mischungen aus Alkyl- und/oder Alkenyloligoglykosiden und Esterquats zur Herstellung von ausgewählten kosmetischen Zubereitungen.

### Stand der Technik

Alkyloligoglucoside stellen nichtionische Tenside dar, die vollständig auf der Grundlage nachwachsender Rohstoffe - Zucker bzw. Stärke und Fettalkohol - aufgebaut sind. Neben ihren hervorragenden anwendungstechnischen Eigenschaften zeichnen sie sich durch eine besonders vorteilhafte ökotoxikologische Verträglichkeit aus, die sie für den Einsatz in einer Vielzahl von Anwendungsfeldem prädestiniert. Auch Mischungen von Alkyloligoglucosiden mit anderen, insbesondere anionischen Tensiden sind aus einer Vielzahl von Publikationen bekannt, von denen stellvertretend die Europäische Patentschrift **EP-B1-0070074** (Procter & Gamble) genannt werden soll. Detergensgemische, die neben Alkyloligoglucosiden kationische Tenside enthalten, werden ebenfalls in einer Reihe von Druckschriften beschrieben. In der **EP-B1-0094118** (Procter & Gamble) werden beispielsweise Zusammensetzungen für phosphatarme Waschmittel offenbart, die neben C₁₂-C₁₈-Alkyloligoglucosiden, Fetalkoholpolyglycolether und quartäre Ammoniumverbindungen enthalten. Gegenstand der Patentanmeldungen **EP-A1-0214285, EP-A1-0246246** (Staley Manuf.Co.) und **WO 87/02050** sind flüssige Desinfektionsmittel bzw. Feinwaschmittel enthaltend Alkyloligoglucoside und quartäre Ammoniumverbindungen. Aus der **DE-A1-3702287** (Colgate) sind schließlich Flüssigwaschmittel bekannt, die neben Alkyloligoglucosiden und quartären Ammoniumverbindungen noch weitere anionische und nichtionische Tenside enthalten. Obschon die Mittel des Stands der Technik in der Regel zufriedenstellende anwendungstechnische Ergebnisse liefern, ist ihre biologische Abbaubarkeit doch im Hinblick auf die ihnen gemeinsame Komponente - die quartären Ammoniumverbindungen (QAV's) - für eine Reihe von Anwendungen nicht ausreichend.

Die Aufgabe der Erfindung bestand somit darin, neue Mittel auf Basis von Alkyloligoglucosiden und kationischen Tensiden zur Verfügung zu stellen, die frei von den geschilderten Nachteilen sind.

### Beschreibung der Erfindung

Gegenstand der Erfindung ist die Verwendung von Detergensgemischen, enthaltend
(a) Alkyl- und/oder Alkenyloligoglykoside der Formel **(I)**,

   **R**^{**1**}**O-[G]**_{**p**} **(I)**

   in der R¹ für Alkyl- oder Alkenylreste mit bis zu 22 Kohlenstoffatomen, [G] für einen Zuckerrest mit 5 oder 6 Kohlenstoffatomen und p für Zahlen (1 bis) 10 steht, und
(b) Esterquats der Formel **(II)**, 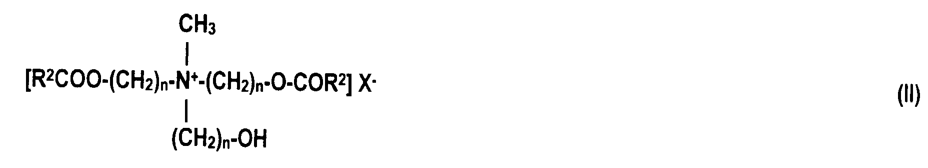 in der R²CO für einen aliphatischen Acylrest mit 12 bis 22 Kohlenstoffatomen und 0, 1, 2 oder 3 Doppelbindungen, n für 2 oder 3 und X für Halogenid, Methosulfat oder Methophosphat steht,
zur Herstellung von Produkten zur Haar- und Körperpflege.

Überraschenderweise wurde gefunden, daß die erfindungsgemäß zu verwendenden Detergensgemische nicht nur über hervorragende anwendungstechnische Eigenschaften verfügen, mit denen sie Produkten des Stands der Technik in vielen Fällen überlegen sind, sondern auch vollständig biologisch abbaubar und toxikologisch unbedenklich sind. Die Erfindung schließt die Erkenntnis ein, daß die Alkyl-und/oder Alkenyloligoglykoside die Solubilisierung der Esterquats in den unterschiedlichsten Medien unterstützen und insbesondere die Bildung von schwerlöslichen Salzen zwischen den Esterquats und gegebenenfalls mitverwendeten anionischen Tensiden weitgehend verhindem.

### Alkyl- und/oder Alkenyloligoglykoside

Alkyl- und oder Alkenyloligoglykosiden stellen bekannte Stoffe dar, die nach den einschlägigen Methoden der präparativen organischen Chemie erhalten werden können. Ein Verfahren zu ihrer Herstellung stellt beispielsweise die sauer katalysierte Acetalisierung von Glucose mit Fettalkoholen dar. Stellvertretend für das umfangreiche Schrifttum sei auf die Europäische Patentanmeldung **EP-A1-0301298** verwiesen. Bevorzugt sind Alkyl- und/oder Alkenyloligoglykoside, die sich von Aldosen bzw. Ketosen und wegen ihrer leichten Verfügbarkeit insbesondere von der Glucose ableiten. Die bevorzugten Alkyloligoglykoside sind somit die Alkyloligo**glucoside**. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyl-und/oder Alkenyloligoglykosid eine analytisch ermittelte rechnerische Größe, die meistens eine gebrochene Zahl darstellt. Bevorzugt sind Alkyl- und/oder Alkenyloligoglykoside mit einem mittleren Oligomerisierungsgrad p von 1,1 bis 3,0; besonders bevorzugt sind solche Alkyl- und/oder Alkenyloligoglykoside, deren Oligomerisierungsgrad kleiner als 1,7 ist und insbesondere zwischen 1,2 und 1,4 liegt. Der Rest R¹ kann sich von gesättigten und/oder ungesättigten primären Alkoholen mit bis zu 22, vorzugsweise 8 bis 10 bzw. 12 bis 18 Kohlenstoffatomen ableiten. Typische Beispiele sind Caprinalkohol, 2-Ethylhexanol, Caprylalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Arachylalkohol, Gadoleylalkohol, Behenylalkohol und Erucylalkohol sowie technische Schnitte, die diese Alkohole in unterschiedlichen Mengen enthalten können. Bevorzugt werden Alkyl- und/oder Alkenyloligoglykoside der Formel **(I)** einsetzt, in der R¹ für Alkylreste mit 8 bis 18 Kohlenstoffatomen, G für einen Glucoserest und p für Zahlen von 1 bis 3 steht.

### Esterquats

Unter Esterquats sind technische quaternierte Di-Fettsäuretrialkanolaminester-Salze zu verstehen, die nach den einschlägigen Methoden der präparativen organischen Chemie zugänglich sind. In diesem Zusammenhang sei auf den Übersichtsartikel von R.Puchta in **C.R. 3. CESIO, 1992, S.122f**. verwiesen. Zur Herstellung der Esterquats geht man üblicherweise von Fettsäuren aus, die im ersten Schritt mit Trialkanolaminen, wie beispielsweise Triethanol- oder Tripropanolamin verestert werden. Der gebildete Difettsäureester kann anschließend in an sich bekannter Weise beispielsweise mit Methylchlorid oder Dimethylsulfat quaterniert werden. Da es sich um technische Produkte handelt, sind in den Esterquats stets quatemierte Mono- und Triester als Nebenprodukte enthalten. Typische Beispiele für die Fettsäurekomponenten dieser Verbindungen sind Capronsäure, Caprylsäure, Caprinsäure, Laurinsäure, Myristinsäure, Palmitinsäure, Stearinsäure, Ölsäure, Elaidinsäure, Petroselinsäure, Linolsäure, Linolensäure, Arachinsäure, Gadoleinsäure, Behensäure und Erucasäure sowie deren technische Mischungen, wie sie beispielsweise bei der Spaltung von pflanzlichen Ölen oder tierischen Fetten anfallen. Bevorzugt sind Esterquats auf der Basis von Stearin- bzw. gehärteter Talgfettsäure in Form ihrer Chloride oder Methosulfate.

### Weitere Tenside

Neben den Alkyl- und/oder Alkenyloligoglykosiden und des Esterquats können die erfindungsgemäßen Detergensgemische noch weitere anionische, nichtionische sowie amphotere bzw. zwitterionische Tenside enthalten, z. B:
(a) **Anionische Tenside:** Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate (mit konventioneller und eingeengter Homologenverteilung), Glycerinethersulfate, Hydroxymischethersulfate, Monoglycerid(ether)sulfate, Fettsäureamid(ether)sulfate, Sulfosuccinate, Sulfosuccinamate, Sulfotriglyceride, Ethercarbonsäuren, Alkyloligoglucosidsulfate und/oder Alkyl(ether)phosphate.
(b) **Nichtionische Tenside**: Fettalkoholethoxylate (mit konventioneller und eingeengter Homologenverteilung), Polyolfettsäureester, Sorbitanester und/oder Polysorbate.
(c) **Amphotere bzw. zwitterionische Tenside**: Alkylamidobetaine, Aminopropionate, Aminoglycinate, Imidazoliniumbetaine und/oder Sulfobetaine.

Das Gewichtsverhältnis von Alkyl- und/oder Alkenyloligoglykosiden und Esterquats kann 5 : 95 bis 95 : 5, vorzugsweise 10 : 90 bis 90 : 10 und insbesondere 30 : 70 bis 70 : 30 betragen. Der Anteil weiterer Tenside an den Detergensgemischen kann dem gegenüber 1 bis 90, vorzugsweise 10 bis 80 und insbesondere 50 bis 70 Gew.-% - bezogen auf das Gemisch - ausmachen. Zur Herstellung der erfindungsgemäßen Detergensgemische ist es ausreichend, die Komponenten, gegebenenfalls unter Erwärmung auf 30 bis 40°C, zu vermischen und - falls erforderlich - zu homogenisieren. Dabei ist sowohl möglich, von Konzentraten auszugehen, die mit Wasser auf eine Anwendungskonzentration von 1 bis 50, vorzugsweise 15 bis 30 Gew.-% verdünnt werden oder gleich verdünnte, wäßrige Ausgangsstoffe einzusetzen. In jedem Fall handelt es sich um einen rein mechanischen Vorgang; eine chemische Reaktion findet nicht statt.

### Gewerbliche Anwendbarkeit

Die erfindungsgemäßen Detergensgemische zeichnen sich durch ausgezeichnete Wasch- und Reinigungsleistung, Haaravivage, antistatischer Ausrüstung von Haaren und Fasern und Kämmbarkeitsverbesserung sowie ökotoxikologische Verträglichkeit aus.

### Beispiele

Typische Beispiele für Zubereitungen, die unter Verwendung der erfindungsgemäßen Detergensgemische hergestellt werden können, sind Haarshampoos, Haarspülungen oder Schaumbäder, die jeweils 10 bis 30 Gew.-% - bezogen auf das Mittel - einer Mischung von Alkyl- und/oder Alkenyloligoglykosiden und Esterquats sowie gegebenenfalls weitere Tenside und übliche Hilfs- und Zusatzstoffe enthalten können. Die Zubereitungen können als Hilfs- und Zusatzstoffe beispielsweise Emulgatoren, Ölkomponenten, Fette und Wachse, Verdickungsmittel, Überfettungsmittel, biogene Wirkstoffe, Filmbildner, Duftstoffe, Farbstoffe, Perlglanzmittel, Konservierungsmittel und pH-Regulatoren enthalten.

Übliche **Ölkomponenten** sind Substanzen wie Paraffinöl, Pflanzenöle, Fettsäureester, Squalan und 2-Octyldodecanol, während als **Fette und Wachse** beispielsweise Walrat, Bienenwachs, Montanwachs, Paraffin und Cetylstearylalkohol Verwendung finden. Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid. Unter **biogenen Wirkstoffen** sind beispielsweise Pflanzenextrakte, Eiweißhydrolysate und Vitaminkomplexe zu verstehen. Gebräuchliche Filmbildner sind beispielsweise Polyvinylpyrrolidon, Vinylpyrrolidon-Vinylacetat-Copolymerisate, Polymere der Acrylsäurereihe, quatemäre Cellulosederivate und ähnliche Verbindungen. Als **Konservierungsmittel** eignen sich beispielsweise Formaldehydlösung, p-Hydroxybenzoat oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984,** zusammengestellt sind. Diese Farbstoffe werden üblicherweise in Konzentrationen von 0,001 bis 0,1 Gew.-%, bezogen auf die gesamte Mischung, eingesetzt.

## Patentansprüche

1. Verwendung von Detergensgemischen, enthaltend
(a) Alkyl- und/oder Alkenyloligoglykoside der Formel **(I)**,
**R**^{**1**}**O-[G]**_{**p**} **(I)**
in der R¹ für Alkyl- oder Alkenylreste mit bis zu 22 Kohlenstoffatomen, [G] für einen Zuckerrest mit 5 oder 6 Kohlenstoffatomen und p für Zahlen von 1 bis 10 steht, und
(b) Esterquats der Formel **(II)**, 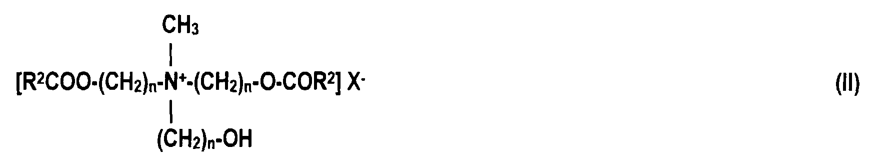 in der R²CO für einen aliphatischen Acylrest mit 12 bis 22 Kohlenstoffatomen und 0, 1, 2 oder 3 Doppelbindungen, n für 2 oder 3 und X für Halogenid, Methosulfat oder Methophosphat steht,
zur Herstellung von Produkten zur Haar- und Körperpflege.

2. Verwendung nach Anspruch 1, **dadurch gekennzeichnet**, daß man die Detergensgemische zusammen mit anionischen, nichtionischen und/oder amphoteren bzw. zwitterionischen Tensiden einsetzt.

3. Verwendung nach Anspruch 2, **dadurch gekennzeichnet**, daß man die Detergensgemische zusammen mit anionischen Tensiden einsetzt, die ausgewählt sind aus der Gruppe, die von Alkylbenzolsulfonaten, Alkansulfonaten, Olefinsulfonaten, Alkylethersulfonaten, Glycerinethersulfonaten, α-Methylestersulfonaten, Sulfofettsäuren, Alkylsulfaten, Fettalkoholethersulfaten, Glycerinethersulfaten, Hydroxymischethersulfaten, Monoglycerid(ether)sulfaten, Fettsäureamid(ether)sulfaten, Sulfosuccinaten, Sulfosuccinamaten, Sulfotriglyceriden, Ethercarbonsäuren, Alkyloligoglucosidsulfaten und Alkyl(ether)phosphaten gebildet wird.

4. Verwendung nach Anspruch 2, **dadurch gekennzeichnet**, daß man die Detergensgemische zusammen mit nichtionischen Tensiden einsetzt, die ausgewählt sind aus der Gruppe, die ausgewählt sind aus der Gruppe, die von Fettalkoholethoxylaten, Polyolfettsäureestern, Sorbitanestern und Polysorbaten gebildet wird.

5. Verwendung nach Anspruch 2, **dadurch gekennzeichnet**, daß man die Detergensgemische zusammen mit amphoteren bzw. zwitterionischen Tensiden einsetzt, die ausgewählt sind aus der Gruppe, die von Alkylamidobetainen, Aminopropionaten, Aminoglycinaten, Imidazoliniumbetainen und Sulfobetainen gebildet wird.

6. Verwendung nach den Ansprüchen 1 bis 5, **dadurch gekennzeichnet**, daß man die Alkyl-und/oder Alkenyloligoglykosiden und Esterquats im Gewichtsverhältnis 5 : 95 bis 95 : 5 einsetzt.

## Claims

1. The use of detergent mixtures containing
(a) alkyl and/or alkenyl oligoglycosides corresponding to formula (I):
R¹-O-[G]ₚ (I)
in which R¹ represents alkyl or alkenyl radicals containing up to 22 carbon atoms, [G] is a sugar unit containing 5 or 6 carbon atoms and p is a number of 1 to 10, and
b) esterquats corresponding to formula (II): 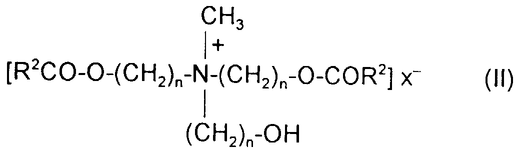 in which R²CO is an aliphatic acyl radical containing 12 to 22 carbon atoms and 0, 1, 2 or 3 double bonds, n = 2 or 3 and X stands for halide, methosulfate or methophosphate,
for the production of hair-care and body-care products.

2. The use claimed in claim 1, characterized in that the detergent mixtures are used together with anionic, nonionic and/or amphoteric or zwitterionic surfactants.

3. The use claimed in claim 2, characterized in that the detergent mixtures are used together with anionic surfactants selected from the group consisting of alkylbenzene sulfonates, alkanesulfonates, olefin sulfonates, alkyl ether sulfonates, glycerol ether sulfonates, α-methyl ester sulfonates, sulfofatty acids, alkyl sulfates, fatty alcohol ether sulfates, glycerol ether sulfates, hydroxy mixed ether sulfates, monoglyceride (ether) sulfates, fatty acid amide (ether) sulfates, sulfosuccinates, sulfosuccinamates, sulfotriglycerides, ether carboxylic acids, alkyl oligoglucoside sulfates and/or alkyl (ether) phosphates.

4. The use claimed in claim 2, characterized in that the detergent mixtures are used together with nonionic surfactants selected from the group consisting of fatty alcohol ethoxylates, polyol fatty acid esters, sorbitan esters and polysorbates.

5. The use claimed in claim 2, characterized in that the detergent mixtures are used together with amphoteric or zwitterionic surfactants selected from the group consisting of alkyl amidobetaines, aminopropionates, aminoglycinates, imidazolinium betaines and sulfobetaines.

6. The use claimed in claims 1 to 5, characterized in that the ratio by weight of alkyl and/or alkenyl oligoglucosides to esterquats is 5:95 to 95:5.

## Revendications

1. Utilisation de mélanges de détergents, contenant :
(a) des alcoyl- et/ou alcényloligoglycosides de formule (I) :
R¹O-[G]ₚ (I)
dans laquelle :
R¹ représente un radical alcoyle ou alcényle, ayant jusqu'à 22 atomes de carbone,
[G] représente un reste sucre ayant 5 ou 6 atomes de carbone, et
p représente un nombre compris dans l'intervalle allant de 1 à 10, et
(b) des esterquats de formule (II) : 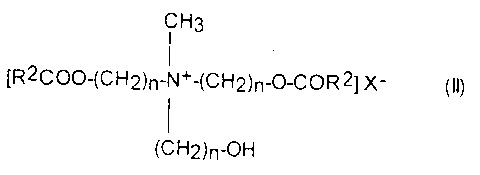 dans laquelle :
R²CO représente un radical acyle aliphatique ayant 12 à 22 atomes de carbone et 0, 1, 2 ou 3 doubles liaisons,
n représente 2 ou 3, et
X représente un halogénure, un méthylsulfate ou un méthylphosphate,
pour la préparation de produits de soin pour les cheveux ou le corps.

2. Utilisation suivant la revendication 1, caractérisée en ce que l'on met en oeuvre les mélanges de détergents avec des tensioactifs anioniques, non ioniques et/ou amphotères ou zwittérioniques.

3. Utilisation suivant la revendication 2, caractérisée en ce que l'on met en oeuvre les mélanges de détergents avec des tensioactifs anioniques qui sont choisis dans le groupe qui est formé des benzènesulfonates d'alcoyle, alcanesulfonates, oléfinesulfonates, alcoyléthersulfonates, glycérineéthersulfonates; -sulfonates d'esters de méthyl sulfonates d'acidesgras sulfogras, sulfates d'alcoyle, éthersulfates d'alcool gras, éthersulfates de glycérine, éthersulfates de composés hydroxylés, (éther)sulfates de monoglycéride, (éther)sulfates d'amide d'acide gras, sulfosuccinates, sulfosuccinamates, sulfotriglycérides, acides éthercarboxyliques, sulfates d'alcoyloligoglucoside et/ou (éther)phosphates d'alcoyle.

4. Utilisation suivant la revendication 2, caractérisée en ce que l'on met en oeuvre les mélanges de détergents avec des tensioactifs non ioniques qui sont choisis dans le groupe qui est formé des éthoxylates d'alcool gras, esters d'acide gras de polyol, esters de sorbitane et/ou polysorbates.

5. Utilisation suivant la revendication 2, caractérisée en ce que l'on met en oeuvre les mélanges de détergents avec des tensioactifs amphotères ou zwittérioniques qui sont choisis dans le groupe qui est formé des alcoylamidobétaïnes, aminopropionates, aminoglycinates, bétaïnes d'imidazolinium et/ou sulfobétaïnes.

6. Utilisation suivant les revendications 1 à 5, caractérisée en ce que l'on met en oeuvre les alcoyl-et/ou alcényloligoglycosides et esterquats suivant un rapport pondéral compris dans l'intervalle allant de 5:95 à 95:5.
